# EUROPEAN PATENT SPECIFICATION

(11) **EP 2 380 581 B1**
(45) Date of publication and mention of the grant of the patent: **26.06.2019**
(21) Application number: 09834346.0
(22) Date of filing: 14.12.2009
(51) Int. Cl.: A23L 33/18, A23L 33/19, A61K 38/01, A23L 2/66, A61P 21/06, C12P 21/06, A23J 3/34, A23K 20/147, A23K 50/40, A23C 9/152, A23C 21/02

(54) **MUSCLE-BUILDING AGENT**
MUSKELAUFBAUPRÄPARAT
AGENT DE DÉVELOPPEMENT MUSCULAIRE

(30) Priority: 24.12.2008 JP 2008328085
(43) Date of publication of application: 26.10.2011
(73) Proprietor: MEGMILK SNOW BRAND CO., LTD., Higashi-ku, Sapporo-shi Hokkaido 0650043 (JP)
(72) Inventor: NAITO, Hisashi, Chiba 270-1606 (JP); MIURA, Susumu, Sapporo-shi Hokkaido 065-0043 (JP)
(74) Representative: Wakerley, Helen Rachael
(86) International application number: PCT/JP2009/006845
(87) International publication number: WO 2010/073531

(56) References cited:
- WO-A1-2007/123200
- WO-A1-2007/143794
- WO-A1-2007/143794
- WO-A1-2008/111562
- WO-A1-2008/111562
- JP-A- 4 112 753
- JP-A- 4 112 753
- JP-A- 2004 182 630
- JP-T- 2004 506 437
- US-A1- 2002 044 988
- US-A1- 2005 164 340

## Description

### TECHNICAL FIELD

The present invention relates to a muscle-building agent which exhibits excellent muscle strength-enhancing effect and effect on early recovery from muscle atrophy due to a long inactive or weightless state, the agent having low bitterness, high stability and safety.

### BACKGROUND ART

A long inactive or weightless state causes various physiological changes (e.g., weight loss, bone decalcification, and skeletal muscle atrophy) of a living body. For example, a long cast immobilization state for treating a fracture or the like, or a long bedridden state, causes a significant decrease in muscle mass as compared with healthy person. It is known that an astronaut who has returned to the earth after a long stay in space cannot stand due to a decrease in muscle mass. In order to deal with the above problems, attempts have been made to enable prevention of, or early recovery from, skeletal muscle atrophy using a dietary ingredient (particularly a protein and an amino acid nutrient). For example, Non-patent Document 1 discloses an effect of reducing skeletal muscle atrophy due to a long weightless state using a soybean protein as a dietary protein source, and suggests that a decrease in abdominal muscle mass is suppressed by intake of a soybean protein.

It is said that when an athlete or the like has performed intense training, the muscle mass adaptively increases due to exercise load irrespective of the nutrient profile. However, deficiency of amino acids that increases the muscle mass may suppress muscle synthesis. Therefore, an effect of intake of proteins or peptides on synthesis of muscle proteins during training has also been studied. For example, Non-patent Document 2 discloses an effect of intake of a soybean peptide on synthesis and maintenance of the muscle of a mouse during long-term exercise, and reports that intake of a soybean peptide increased the weight of the abdominal muscles and the musculus quadriceps.

However, since a soybean peptide has peculiar bitterness, and cloudiness occurs when dispersing a soybean peptide in water, a soybean peptide is limitedly used for a desired food, drink, or the like. In particular, a soybean peptide cannot be used for a product for which transparency is desired.

A whey protein contained in cow's milk has a high content of branched-chain amino acids that is involved in muscle synthesis as compared with a soybean protein, and has high net protein utilization (NPU). However, an effect of intake of a whey protein or a whey peptide (i.e., whey protein hydrolyzate) on an increase in muscle mass has not been reported systematically.

A milk protein hydrolyzate has been used for various products in order to prevent food allergy caused by cow's milk or a dairy product. It is considered that a whey protein contained in cow's milk functions as an allergen, differing from a protein contained in mother's milk. This problem may be solved by hydrolyzing a whey protein using protease (see Patent Documents 1 and 2, for example).

For example, when producing a whey protein hydrolyzate by performing a protease treatment, inactivating the protease with heating, and then performing an additional protease treatment, there are a lot of problems, for example, 1) it is difficult to perform a protease treatment on a precipitate and an aggregate of a whey protein that is purified after inactivation, so that a decrease in antigenicity and an increase in yield may not be achieved, and 2) the yield may decrease when whey protein is heated (at 90°C for 10 minutes or more) before a protease treatment.
Patent Document 1: JP-A-2-002319
Patent Document 2: JP-A-2-138991

Non-patent Document 1: Tada et al., Soy Protein Research, Vol. 2, pp. 112-117 (1999)
Non-patent Document 2: Fushiki et al., Soy Protein Research, Vol. 16, pp. 1-3 (1995)

Whey protein hydrolyzates for other treatments and purposes have been disclosed by the applicant or its predecessor in documents JP 4112753A and WO 2008/111562 A1. Another disclosure by of whey protein hydrolysis is in US 2005/164340 A1.

The use of whey proteins in general in the context of muscle synthesis is disclosed in US 2002/0044988 A1 and in muscle recovery after exercise in WO 2007/143794 A1.

### DISCLOSURE OF THE INVENTION

### PROBLEMS TO BE SOLVED BY THE INVENTION

The invention is defined by the claims. An object of the invention is to provide a muscle-building agent comprising a whey protein hydrolyzate that exhibits a muscle mass-increasing effect.

Another object of the invention is to provide a muscle-building food that exhibit a muscle-building effect, are very safe, and can be taken orally (i.e., good flavor).

### MEANS FOR SOLVING THE PROBLEMS

In order to achieve the above objects, the inventors of the invention conducted extensive studies on a whey protein hydrolyzate that is obtained by hydrolyzing and thermally denaturing a whey protein using a heat-resistant protease under given conditions, and found that a whey protein hydrolyzate having a molecular weight distribution of 10 kDa or less, a main peak of 200 Da to 3 kDa, an average peptide length (APL) of 2 to 8, a free amino acid content of 20% or less of all components, and antigenicity equal to or less than 1/100,000th of that of β-lactoglobulin, exhibits a muscle-building effect that achieves the above objects.

Specifically, the invention provides the following.
(1) A whey protein hydrolyzate for use in the treatment of muscle atrophy, the whey protein hydrolyzate having (1) a molecular weight distribution of 10 kDa or less and a main peak of 200 Da to 3 kDa, (2) an average peptide length (APL) of 2 to 8, (3) a free amino acid content of 20% or less, and (4) antigenicity equal to or less than 1/100,000th of that of β-lactoglobulin, and wherein the whey protein hydrolyzate is for administration in an amount of 10g/day or more.
(2) The whey protein hydrolyzate for use in the treatment according to (1), wherein the whey protein hydrolyzate is obtained by hydrolyzing and thermally denaturing a whey protein at pH 6 to 10 at 50 to 70°C using a heat-resistant protease to obtain a mixture, and heating the mixture to inactivate the protease.
(3) The whey protein hydrolyzate for use in the treatment according to (1), wherein the whey protein hydrolyzate is obtained by performing a preliminary hydrolysis step that hydrolyzes a whey protein at pH 6 to 10 at 20 to 55°C using a protease to obtain a mixture, heating the mixture to 50 to 70°C, hydrolyzing and thermally denaturing an unhydrolyzed whey protein included in the mixture at pH 6 to 10 at 50 to 70°C using a heat-resistant protease, and heating the mixture to inactivate the protease.
(4) The muscle-building agent according to (1), wherein the whey protein hydrolyzate is obtained by performing a preliminary hydrolysis step that hydrolyzes a whey protein at pH 6 to 10 at 20 to 55°C using a protease to obtain a mixture, heating the mixture to 50 to 70°C, hydrolyzing and thermally denaturing an unhydrolyzed whey protein included in the mixture at pH 6 to 10 at 50 to 70°C using a heat-resistant protease, heating the mixture to inactivate the protease, removing an unhydrolyzed protein from the mixture using an ultrafiltration membrane having a molecular weight cut-off of 1 to 20 kDa, and removing a low-molecular-weight fraction from the mixture using a microfiltration membrane having a molecular weight cut-off of 100 to 500 Da.

The whey protein hydrolyzate may be in the form of a muscle-building food or drink, a muscle-building nutrient composition, or a muscle-building feed.

### EFFECTS OF THE INVENTION

The above whey protein hydrolyzate exhibits a remarkable muscle synthesis effect, and is useful for recovery from muscle atrophy. The disclosure also relates to recovery of muscle damaged through intense training.

It was confirmed that the whey protein hydrolyzate used as an active ingredient has antigenicity equal to or less than 1/100,000th of that of β-lactoglobulin and whey protein as described later. Therefore, the whey protein hydrolyzate is very safe.

The solubility of the whey protein hydrolyzate in water can be improved by filtering the whey protein hydrolyzate using an ultrafiltration (UF) membrane and a microfiltration (MF) membrane.

Since the above muscle-building agent is produced using a whey protein as a raw material, the muscle-building agent can be easily and economically produced.

### EXEMPLARY EMBODIMENTS OF THE INVENTION

A muscle-building agent for use in the treatment of muscle atrophy comprises a whey protein hydrolyzate that has a muscle-building effect, and is obtained by hydrolyzing and thermally denaturing a whey protein at pH 6 to 10 at 50 to 70°C using a heat-resistant protease to obtain a mixture, and heating the mixture to inactivate the protease. The yield of the whey protein hydrolyzate can be improved by hydrolyzing the whey protein at pH 6 to 10 at 20 to 55°C using a protease, and then quickly hydrolyzing the whey protein under the above conditions without cooling the whey protein.

The muscle-building effect can be improved by concentrating the whey protein hydrolyzate obtained as described above using an ultrafiltration (UF) membrane having a molecular weight cut-off of 1 to 20 kDa, and preferably 2 to 10 kDa, and a microfiltration membrane having a molecular weight cut-off of 100 to 500 Da, and preferably 150 to 300 Da. This also makes it possible to reduce bitterness, and improve transparency.

The term "whey protein" used herein refers to whey of a mammal such as a cow, a buffalo, a goat, or a human, or an aggregate, a powder or a purified product thereof. The whey protein is used in a state of an aqueous solution in an enzyme reaction.

The pH of the solution is adjusted to 6 to 10. The whey protein solution normally has a pH within the above range. When it is necessary to adjust the pH of the solution, the pH of the solution is adjusted to 6 to 10 using an acid solution (e.g., hydrochloric acid, citric acid, or lactic acid) or an alkali solution (e.g., caustic soda, calcium hydroxide, or sodium phosphate). The solution is then heated to 50 to 70°C. It is preferable to add the heat-resistant protease before the heating step to 50 to 70°C so that hydrolysis also occurs during heating before the temperature reaches to 50 °C to 70°C from the viewpoint of the yield.

While the optimum temperature for a normal protease is 40°C or less, the optimum temperature for a heat-resistant protease is 45°C or more. An arbitrary heat-resistant protease having an optimum temperature of 45°C or more may be used. Examples of such a heat-resistant protease include papain, Protease S (trade name), Proleather (trade name), Thermoase (trade name), Alcalase (trade name), Protin A (trade name), and the like. It is preferable to use a heat-resistant protease that has a remaining activity of 10% or more when heated at 80°C for 30 minutes. It is effective to use a plurality of proteases in combination. The reaction time is preferably about 30 minutes to 10 hours.

After denature of the protease, the reaction solution is then heated to inactivate the protease. The protease may be inactivated by heating the reaction solution at 100°C or more for 10 seconds or more.

After the inactivation of protease, the reaction solution is centrifuged, and the supernatant liquid is collected. The supernatant liquid is then dried to obtain a powdery product. A precipitate that occurs due to centrifugation is less hypoallergenic as compared with the supernatant liquid, and is preferably removed. Note that the reaction solution may be dried and used directly insofar as antigenicity problem does not occur.

It was confirmed that a whey protein hydrolyzate obtained by the above method has antigenicity equal to or less than 1/100,000th of that of β-lactoglobulin and the whey protein when measured by inhibition ELISA (see the Japanese Journal of Pediatric Allergy and Clinical Immunology, 1, 36 (1987)). Therefore, the whey protein hydrolyzate is very safe. Since an aqueous solution of the whey protein hydrolyzate is transparent, and has a bitterness level of about 2, the whey protein hydrolyzate may be used for an arbitrary product.

The whey protein hydrolyzate according to one embodiment of the invention may be used directly, or may be formed into a powdered drug, granules, a tablet, a capsule, a drinkable preparation, or the like by a normal method. Also a whey protein hydrolyzate obtained using an ultrafiltration (UF) membrane or a microfiltration (MF) membrane may be used directly as a muscle-building agent, or may be used after drying. The whey protein hydrolyzate may also be formed into a preparation by a normal method.

The preparation may be added to a nutrient preparation, food or drink (e.g., yogurt, milk-based drink, or wafer), feed, a supplement, or the like.

The amount of the whey protein hydrolyzate in a muscle-building food or drink, a muscle-building nutrient composition, or a muscle-building feed is not particularly limited. According to the invention, an adult takes the whey protein hydrolyzate in an amount of 10 g/day or more, and preferably 20 g/day or more.

The whey protein hydrolyzate as an active ingredient may be mixed with an appropriate auxiliary agent, and formed into an oral preparation (muscle-building agent).

The invention is further described below by way of examples, comparative examples, and test examples. Note that the following examples are given for illustrative purposes, and should not be construed as limiting the invention.

In the examples, transparency and bitterness were evaluated by the following methods.
Transparency evaluation method: A 1% whey protein hydrolyzate solution was prepared, and the absorbance at 650 nm was measured.
Bitterness evaluation method: A 10% whey protein hydrolyzate solution was prepared. The bitterness of the solution was evaluated using quinine hydrochloride (bitter substance). If the bitterness level is 2 or less as shown in Table 1, the whey protein hydrolyzate can be used for food, drink, or the like.

**TABLE 1**

| Quinine hydrochloride concentration | Bitterness level |
|---|---|
| 0.004% | 1 (Low) |
| 0.010% | 2 |
| 0.020% | 3 (High) |

### Example 1

Papain (50 U/g·whey protein) and Proleather (manufactured by Amano Enzyme Inc.) (150 U/g·whey protein) were added to 11 of a 10% whey protein aqueous solution. After adjusting the pH of the mixture to 8, the whey protein was hydrolyzed and denatured at 55°C for 6 hours. The reaction solution was heated at 100°C for 15 seconds or more to inactivate the proteases. The reaction solution was then centrifuged, and the supernatant liquid was collected. The supernatant liquid was then dried to obtain a whey protein hydrolyzate.

The obtained whey protein hydrolyzate had a molecular weight distribution of 10 kDa or less, a main peak of 1.3 kDa, an APL of 7.2, and a free amino acid content of 18.9% of all components.

The whey protein hydrolyzate had antigenicity equal to or less than 1/100,000th of that of β-lactoglobulin (measured by inhibition ELISA). The yield (i.e., the ratio (%) of the dry weight of the supernatant liquid obtained by centrifugation of the enzyme reaction solution to the dry weight of the raw material) was 80.3%, and the bitterness level was 2.

The amino acid composition of the whey protein hydrolyzate is shown in Table 2.

The whey protein hydrolyzate thus obtained can be used directly as the muscle-building agent.

Since the aqueous solution of the whey protein hydrolyzate obtained in Example 1 was transparent and had a bitterness level of about 2, the whey protein hydrolyzate can be easily formed into a preparation, food, drink, or the like.

**TABLE 2**

| Amino acid | % |
|---|---|
| Aspartic acid | 10.6 |
| Threonine | 5.0 |
| Serine | 4.6 |
| Glutamic acid | 17.4 |
| Proline | 3.6 |
| Glycine | 1.8 |
| Alanine | 5.2 |
| Valine | 5.4 |
| Cystine | 2.5 |
| Methionine | 2.2 |
| Isoleucine | 5.7 |
| Leucine | 12.3 |
| Tyrosine | 3.5 |
| Phenylalanine | 3.7 |
| Ammonia | - |
| Lysine | 9.8 |
| Histidine | 1.9 |
| Tryptophan | 2.0 |
| Arginine | 2.7 |

### Example 2

Papain (50 U/g·whey protein) and Proleather (150 U/g·whey protein) were added to 1 1 of a 10% whey protein aqueous solution. After adjusting the pH of the mixture to 8, the whey protein was hydrolyzed at 50°C for 3 hours. The mixture was then heated to 55°C, and the whey protein was denatured and hydrolyzed at 55°C for 3 hours. The mixture was then heated at 100°C for 15 seconds or more to inactivate the proteases. The reaction solution was filtered using a UF membrane having a molecular weight cut-off of 10 kDa (manufactured by STC) and an MF membrane having a molecular weight cut-off of 300 Da (manufactured by STC) to collect a concentrate fraction. The fraction was then dried to obtain a whey protein hydrolyzate.

The obtained whey protein hydrolyzate had a molecular weight distribution of 10 kDa or less, a main peak of 500 Da, an APL of 3.0, and a free amino acid content of 15.2% of all components.

The whey protein hydrolyzate had antigenicity equal to or less than 1/100,000th of that of β-lactoglobulin (measured by inhibition ELISA). The yield was 65.4%, and the bitterness level was 2.

The whey protein hydrolyzate thus obtained can be used directly as the muscle-building agent.

### Comparative Example 1

120 g of a whey protein was dissolved in 1800 ml of purified water, and the pH of the solution was adjusted to 7.0 using a 1M caustic soda solution. The solution was sterilized at 60°C for 10 minutes, and held at 45°C. After the addition of 20 g of Amano A (manufactured by Amano Enzyme Inc.), the mixture was reacted for 2 hours. The mixture was then heated at 80°C for 10 minutes to inactivate the protease, and freeze-dried to obtain a whey protein hydrolyzate.

The hydrolysis rate of the whey protein hydrolyzate was 18%, and the yield was 80.6%.

### Comparative Example 2

120 g of a whey protein was dissolved in 1800 ml of purified water, and the pH of the solution was adjusted to 7.0 using a 1M caustic soda solution. The solution was sterilized at 60°C for 10 minutes, and held at 45°C. After the addition of 20 g of Amano A (manufactured by Amano Enzyme Inc.), the mixture was reacted for 8 hours. The mixture was then heated at 80°C for 10 minutes to inactivate the protease, and freeze-dried to obtain a whey protein hydrolyzate.

The hydrolysis rate of the whey protein hydrolyzate was 30%, and the yield was 80.6%.

### Test Example 1

### (Transparency test)

A 1% aqueous solution of each of the whey protein hydrolyzates obtained in Examples 1 and 2 and Comparative Examples 1 and 2 was prepared, and the absorbance at 650 nm was measured. The results are shown in Table 3.

**TABLE 3**

| Sample | Absorbance (650nm) |
|---|---|
| Whey protein hydrolyzate (Example 1) | 0.008 |
| Whey protein hydrolyzate (Example 2) | 0.004 |
| Comparative Example 1 | 0.064 |
| Comparative Example 2 | 0.018 |

The whey protein hydrolyzates obtained in Examples 1 and 2 had a low absorbance (i.e., high transparency). The whey protein hydrolyzates obtained in Comparative Examples 1 and 2 had a high absorbance (i.e., low transparency) as compared with the whey protein hydrolyzates obtained in Examples 1 and 2. The whey protein hydrolyzate obtained in Example 2 that was subjected to the membrane treatment had a low absorbance (i.e., high transparency) as compared with the whey protein hydrolyzate obtained in Example 1.

### Test Example 2

### (Muscular strength recovery test)

Wistar female rats (5 weeks old) purchased from Japan SLC Inc. were divided into four groups so that the weight of each group was equal at 6 weeks old (n=6 to 7 in each groups, 144±6.8 g).

The rats were arbitrarily fed a standard AIN93 diet in which an unhydrolyzed whey protein, the whey protein hydrolyzate obtained in Example 1, or the whey protein hydrolyzate obtained in Example 2 was used instead of a casein protein. The diet intake was measured every 2 to 3 days.

All of the rats were fed the standard AIN93 diet during a hindlimb suspension period (1 week). After completion of the hindlimb suspension period, the rats were kept in a normal cage for 1 week under a different dietary condition (recovery period). A group that was fed a standard diet and was not subjected to hindlimb suspension was used as a control.

After completion of the recovery period, the soleus muscle (SOL) was removed under anesthesia with pentobarbital sodium (50 mg/kg), and the weight of the muscle proteins was measured.

The measurement results of the relative soleus muscle protein content against body weight are shown in Table 4.

**TABLE 4**

| | SOL content/body weight |
|---|---|
| Control | 0.314±0.038 |
| Standard diet | 0.268±0.024 (b) |
| Whey protein | 0.261±0.022 (b) |
| Whey protein hydrolyzate (Example 1) | 0.293±0.020 (cd) |
| Whey protein hydrolyzate (Example 2) | 0.297±0.019 (cd) |

| | |
|---|---|
| b: A significant difference was observed as compared with the control (p<0.05). c: A significant difference was observed as compared with the standard diet group (p<0.05). d: A significant difference was observed as compared with the whey protein group (p<0.05). | |

Only the standard diet group and the whey protein group showed a significant decrease in muscle weight as compared with the control. The groups that were fed the whey protein hydrolyzate obtained in Example 1 or 2 did not show a significant decrease in muscle weight.

### Example 3

### Preparation of muscle-building nutrient composition (not within the scope of the invention)

50 g of the whey protein hydrolyzate obtained in Example 2 was dissolved in 4950 g of deionized water. The solution was heated to 50°C, and stirred at 6000 rpm for 30 minutes using a TK-homomixer ("TKROBOMICS" manufactured by PRIMIX Corporation) to obtain a whey protein hydrolyzate solution (whey protein hydrolyzate content: 50 g/5 kg). 5.0 kg of casein, 5.0 kg of a soybean protein, 1.0 kg of fish oil, 3.0 kg of perilla (shiso) oil, 18.0 kg of dextrin, 6.0 kg of a mineral mixture, 1.95 kg of a vitamin mixture, 2.0 kg of an emulsifying agent, 4.0 kg of a stabilizer, and 0.05 kg of aroma chemical were added to 5.0 kg of the whey protein hydrolyzate solution. A retort pouch (200 ml) was filled with the mixture. The mixture was then sterilized at 121°C for 20 minutes using a retort sterilizer (class-1 pressure vessel, "RCS-4CRTGN" manufactured by Hisaka Works, Ltd.) to produce 50 kg of a muscle-building nutrient composition of the present invention.

The muscle-building nutrient composition contained the whey protein hydrolyzate in an amount of 100 mg/100 g.

### Example 4

### Preparation of muscle-building drink (not within the scope of the invention)

300 g of a skimmed milk powder was dissolved in 409 g of deionized water. 1 g of the whey protein hydrolyzate obtained in Example 1 was then dissolved in the solution. The resulting solution was heated to 50°C, and stirred at 9500 rpm for 30 minutes using an ultra-disperser ("ULTRA-TURRAX T-25" manufactured by IKA Japan). After the addition of 100 g of maltitol, 2 g of an acidulant, 20 g of reduced starch syrup, 2 g of aroma chemical, and 166 g of deionized water, a glass bottle (100 ml) was charged with the mixture. After sterilization at 90°C for 15 minutes, the bottle was sealed to obtain ten bottles (100 ml) of a muscle-building drink.

The muscle-building drink contained the whey protein hydrolyzate in an amount of 100 mg/100 ml.

### Example 5

### Preparation of muscle-building dog food (not within the scope of the invention)

200 g of the whey protein hydrolyzate obtained in Example 2 was dissolved in 99.8 kg of deionized water. The solution was heated to 50°C, and stirred at 3600 rpm for 40 minutes using a TK-homomixer ("MARK II 160" manufactured by PRIMIX Corporation) to obtain a whey protein hydrolyzate solution (whey protein hydrolyzate content: 2 g/100 g). 12 kg of soybean meal, 14 kg of powdered skim milk, 4 kg of soybean oil, 2 kg of corn oil, 23.2 kg of palm oil, 14 kg of corn starch, 9 kg of flour, 2 kg of bran, 5 kg of a vitamin mixture, 2.8 kg of cellulose, and 2 kg of a mineral mixture were added to 10 kg of the whey protein hydrolyzate solution. The mixture was sterilized at 120°C for 4 minutes to obtain 100 kg of a muscle-building dog food of the present invention.

The muscle-building dog food contained the whey protein hydrolyzate in an amount of 20 mg/100 g.

## Claims

1. A whey protein hydrolyzate for use in the treatment of muscle atrophy, wherein the whey protein hydrolyzate has (1) a molecular weight distribution of 10 kDa or less and a main peak of 200 Da to 3 kDa, (2) an average peptide length (APL) of 2 to 8, (3) a free amino acid content of 20% or less, (4) a branched-chain amino acid content of 20% or more, and (5) antigenicity equal to or less than 1/100,000th of that of β-lactoglobulin, and wherein the whey protein hydrolyzate is for administration in an amount of 10 g/day or more.

2. The whey protein hydrolyzate for use in the treatment according to claim 1, wherein the whey protein hydrolyzate is obtained by performing a hydrolysis step that hydrolyzes and thermally denatures a whey protein at pH 6 to 10 at 50 to 70°C using a heat-resistant protease to obtain a mixture, and an inactivation step that heats the mixture to inactivate the protease.

3. The whey protein hydrolyzate for use in the treatment according to claim 1, wherein the whey protein hydrolyzate is obtained by performing a preliminary hydrolysis step that hydrolyzes a whey protein at pH 6 to 10 at 20 to 55°C using a protease to obtain a mixture, a hydrolysis step that heats the mixture to 50 to 70°C, and hydrolyzes and thermally denatures an unhydrolyzed whey protein included in the mixture at pH 6 to 10 at 50 to 70°C using a heat-resistant protease, and an inactivation step that heats the mixture to inactivate the protease.

4. The whey protein hydrolyzate for use in the treatment according to claim 1, wherein the whey protein hydrolyzate is obtained by performing a preliminary hydrolysis step that hydrolyzes a whey protein at pH 6 to 10 at 20 to 55°C using a protease to obtain a mixture, a hydrolysis step that heats the mixture to 50 to 70°C, and hydrolyzes and thermally denatures an unhydrolyzed whey protein included in the mixture at pH 6 to 10 at 50 to 70°C using a heat-resistant protease, an inactivation step that heats the mixture to inactivate the protease, an ultrafiltration step that filters the mixture using an ultrafiltration membrane having a molecular weight cut-off of 1 to 20 kDa, and a microfiltration step that filters the filtrate obtained by the ultrafiltration step using a microfiltration membrane having a molecular weight cut-off of 100 to 500 Da to obtain an unfiltered fraction.

## Patentansprüche

1. Molkenproteinhydrolysat zur Verwendung bei der Behandlung von Muskelatrophie, wobei das Molkenproteinhydrolysat (1) eine Molekülmassenverteilung von 10 kDa oder weniger und einen Hauptpeak von 200 Da bis 3 kDa, (2) eine durchschnittliche Peptidlänge (APL) von 2 bis 8, (3) einen freien Aminosäuregehalt von 20 % oder weniger, (4) einen Gehalt an verzweigtkettiger Aminosäure von 20 % oder mehr und (5) Antigenität von gleich oder kleiner als 1/100.000tel von der von β-Lactoglobulin hat und wobei das Molkenproteinhydrolysat zur Verabreichung in einer Menge von 10 g/Tag oder mehr vorgesehen ist.

2. Molkenproteinhydrolysat zur Verwendung bei der Behandlung nach Anspruch 1, wobei das Molkenproteinhydrolysat erhalten wird, indem die folgenden Schritte ausgeführt werden: ein Hydrolyseschritt, bei dem ein Molkenprotein bei pH 6 bis 10 und 50 bis 70°C unter Verwendung einer hitzeresistenten Protease hydrolysiert und thermisch denaturiert wird, um ein Gemisch zu erhalten, und ein Inaktivierungsschritt, bei dem das Gemisch zum Inaktivieren der Protease erhitzt wird.

3. Molkenproteinhydrolysat zur Verwendung bei der Behandlung nach Anspruch 1, wobei das Molkenproteinhydrolysat erhalten wird, indem die folgenden Schritte ausgeführt werden: ein einleitender Hydrolyseschritt, bei dem ein Molkenprotein bei pH 6 bis 10 und 20 bis 55°C unter Verwendung einer Protease hydrolysiert wird, um ein Gemisch zu erhalten, ein Hydrolyseschritt, bei dem das Gemisch auf 50 bis 70°C erhitzt wird und ein in dem Gemisch enthaltenes nicht hydrolysiertes Molkenprotein bei pH 6 bis 10 und 50 bis 70°C unter Verwendung einer hitzeresistenten Protease hydrolysiert und thermisch denaturiert wird, und ein Inaktivierungsschritt, bei dem das Gemisch zum Inaktivieren der Protease erhitzt wird.

4. Molkenproteinhydrolysat zur Verwendung bei der Behandlung nach Anspruch 1, wobei das Molkenproteinhydrolysat erhalten wird, indem die folgenden Schritte ausgeführt werden: ein einleitender Hydrolyseschritt, bei dem das Molkenprotein bei pH 6 bis 10 und 20 bis 55°C unter Verwendung einer Protease hydrolysiert wird, um ein Gemisch zu erhalten, ein Hydrolyseschritt, bei dem das Gemisch auf 50 bis 70°C erhitzt wird und ein in dem Gemisch enthaltenes nicht hydrolysiertes Molkenprotein bei pH 6 bis 10 und 50 bis 70°C unter Verwendung einer hitzeresistenten Protease hydrolysiert und thermisch denaturiert wird, ein Inaktivierungsschritt, bei dem das Gemisch zum Inaktivieren der Protease erhitzt wird, ein Ultrafiltrationsschritt, bei dem das Gemisch mit einer Ultrafiltrationsmembran mit einer Molekülmassen-Ausschlussgrenze von 1 bis 20 kDa filtriert wird, und ein Mikrofiltrationsschritt, bei dem das im Ultrafiltrationsschritt erhaltene Filtrat mit einer Mikrofiltrationsmembran mit einer Molekülmassen-Ausschlussgrenze von 100 bis 500 Da filtriert wird, um eine unfiltrierte Fraktion zu erhalten.

## Revendications

1. Hydrolysat de protéine de lactosérum à utiliser dans le traitement d'atrophie musculaire, où l'hydrolysat de protéine de lactosérum a (1) une distribution de poids moléculaire de 10 kDa ou moins et un pic principal de 200 Da à 3 kDa, (2) une longueur moyenne de peptide (APL) de 2 à 8, (3) une teneur en acides aminés libres de 20 % ou moins, (4) une teneur en acides aminés à chaîne ramifiée de 20 % ou plus, et (5) une antigénicité égale à ou de moins de 1/100000^{e} de celle de la β-lactoglobuline, et où l'hydrolysat de protéine de lactosérum est destiné à être administré en une quantité de 10 g/jour ou plus.

2. Hydrolysat de protéine de lactosérum à utiliser dans le traitement selon la revendication 1, où l'hydrolysat de protéine de lactosérum est obtenu en effectuant une étape d'hydrolyse qui hydrolyse et dénature thermiquement une protéine de lactosérum à pH de 6 à 10 à 50 à 70 °C en utilisant une protéase résistante à la chaleur pour obtenir un mélange, et une étape d'inactivation qui chauffe le mélange pour inactiver la protéase.

3. Hydrolysat de protéine de lactosérum à utiliser dans le traitement selon la revendication 1, où l'hydrolysat de protéine de lactosérum est obtenu en effectuant une étape d'hydrolyse préliminaire qui hydrolyse une protéine de lactosérum à pH de 6 à 10 à 20 à 55 °C en utilisant une protéase pour obtenir un mélange, une étape d'hydrolyse qui chauffe le mélange de 50 à 70 °C, et hydrolyse et dénature thermiquement une protéine de lactosérum non hydrolysée incluse dans le mélange à pH de 6 à 10 à 50 à 70 °C en utilisant une protéase résistante à la chaleur, et une étape d'inactivation qui chauffe le mélange pour inactiver la protéase.

4. Hydrolysat de protéine de lactosérum à utiliser dans le traitement selon la revendication 1, où l'hydrolysat de protéine de lactosérum est obtenu en effectuant une étape d'hydrolyse préliminaire qui hydrolyse une protéine de lactosérum à pH de 6 à 10 à 20 à 55 °C en utilisant une protéase pour obtenir un mélange, une étape d'hydrolyse qui chauffe le mélange de 50 à 70 °C, et hydrolyse et dénature thermiquement une protéine de lactosérum non hydrolysée incluse dans le mélange à pH de 6 à 10 à 50 à 70 °C en utilisant une protéase résistante à la chaleur, une étape d'inactivation qui chauffe le mélange pour inactiver la protéase, une étape d'ultrafiltration qui filtre le mélange en utilisant une membrane d'ultrafiltration ayant un seuil de coupure par poids moléculaire de 1 à 20 kDa, et une étape de microfiltration qui filtre le filtrat obtenu par l'étape d'ultrafiltration en utilisant une membrane de microfiltration ayant un seuil de coupure par poids moléculaire de 100 à 500 Da pour obtenir une fraction non filtrée.
